# EUROPEAN PATENT APPLICATION

(11) **EP 4 579 668 A1**
(43) Date of publication of application: **02.07.2025**
(21) Application number: 23857706.8
(22) Date of filing: 23.08.2023
(51) Int. Cl.: G16B 40/00, G16B 50/00, C12N 15/113

(54) **METHOD AND DEVICE FOR EVALUATING EFFICIENCY OF CANDIDATE SHRNA USED FOR RNA INTERFERENCE**

(30) Priority: 23.08.2022 KR 20220105172; 22.08.2023 KR 20230110104
(71) Applicant: IUCF-HYU (Industry-University Cooperation Foundation Hanyang University), Seoul 04763 (KR)
(72) Inventor: NAM, Jin-Wu, Seoul 06009 (KR); PARK, Seokju, Seoul 04792 (KR)
(74) Representative: dompatent von Kreisler Selting Werner - Partnerschaft von Patent- und Rechtsanwälten mbB
(86) International application number: PCT/KR2023/012449
(87) International publication number: WO 2024/043674

(57) **Abstract**

This method for evaluating the efficiency of RNA interference comprises steps in which an evaluation device: receives sequence information about candidate guide RNAs used for RNA interference and information about the amount of pri-shRNA remaining after cleaving by Drosha; inputs, into an evaluation model evaluating the efficiency of shRNAs used for RNA interference, the received guide RNA sequence information and information about the amount of pri-shRNA remaining after cleaving; and evaluates, on the basis of values output by the evaluation model, the efficiency of the shRNAs used for RNA interference.

## Description

### [Technical Field]

The invention described below relates to a device and a method for evaluating the efficiency of candidate short hairpin RNA (shRNA) used for RNA interference.

### [Background Art]

RNA interference refers to artificially controlling the expression of specific genes using short RNA. RNA interference may be achieved using microRNA (miRNA) and small interfering RNA (siRNA).

Short hairpin RNA (shRNA) is one of the RNAs used for RNA interference. shRNA interference refers to RNA interference by shRNA expressed in cells after transforming cells to mimic the maturation pathway of miRNA.

### [Disclosure]

### [Technical Problem]

In order to efficiently control gene expression through the shRNA method, the design of guide RNA used for the shRNA method is important. Various conventional methods of designing guide RNA are known. However, the previously known methods had the problem of using data from siRNA rather than shRNA or using only the sequence information of guide RNA.

The invention described below is directed to providing a technique for evaluating the efficiency of candidate shRNA used for RNA interference by using the sequence information of guide RNA used for the shRNA method, as well as the information about the cleavage efficiency of Drosha determined through the amount of primary shRNA (pri-shRNA).

### [Technical Solution]

A method of evaluating the efficiency of a candidate short hairpin RNA (shRNA) used for RNA interference includes: a step in which an evaluation device receives sequence information about a candidate guide RNA used for RNA interference and information about the amount of primary short hairpin RNA (pri-shRNA) remaining after being cleaved by Drosha; a step in which the evaluation device inputs the received 'sequence information about the guide RNA' and 'information about the amount of pri-shRNA remaining after being cleaved' into an evaluation model evaluating the efficiency of shRNA used for RNA interference; and a step in which the evaluation device evaluates the efficiency of the shRNA used for RNA interference based on values output by the evaluation model.

A device for evaluating the efficiency of a candidate shRNA used for RNA interference includes: an input device receiving sequence information about a candidate guide RNA used for RNA interference and information about the amount of pri-shRNA remaining after being cleaved by Drosha; a storage device storing an evaluation model that evaluates the efficiency of shRNA used for RNA interference, using the guide RNA sequence information and the information about the amount of pri-shRNA remaining after being cleaved, received by the input device; and a computation device inputting the sequence information about the guide RNA and the information about the amount of pri-shRNA remaining after being cleaved, received by the input device, into the evaluation model stored in the storage device, and then evaluating the efficiency of shRNA used for RNA interference according to values output by the evaluation model.

### [Advantageous Effects]

When the invention described below is used, the efficiency of candidate shRNA used for RNA interference can be determined by using not only the sequence information about guide RNA used for shRNA but also the cleavage efficiency information of Drosha. In addition, when the technology described below is used, the efficiency of candidate shRNA can be determined by further using the flanking sequence information of guide RNA. This can also be helpful in designing candidate shRNA used for RNA interference.

The invention described below can also be helpful in developing a therapeutic for a disease using an RNA interference method.

### [Description of Drawings]

FIG. 1 shows an example illustrating the overall process for determining the efficiency of shRNA used for RNA interference.
FIG. 2 shows the experimental results illustrating that the 'amount of pri-shRNA' and the 'efficiency of shRNA' are negatively correlated.
FIG. 3 shows the experimental results illustrating that the 'amount of pri-shRNA' and the 'efficiency of pri-shRNA being cleaved by Drosha' are negatively correlated.
FIG. 4 shows an example illustrating the types and number of pieces of training or test data used for an evaluation model 310.
FIG. 5 shows an example in which the structure of an evaluation model implemented as a learning model is implemented as a convolution neural network (CNN).
FIG. 6 shows the experimental results confirming the performance of an evaluation model 310 according to data that may be further input. FIG. 7 shows an example in which the structure of a prediction model implemented as a learning model is implemented as a CNN.
FIG. 8 shows the experimental results of evaluating the performance of a prediction model.
FIG. 9 shows the experimental results of evaluating the performance of a shRNA efficiency prediction method using an evaluation model and a prediction model.
FIG. 10 shows the experimental results of evaluating the performance of a shRNA efficiency prediction method using an evaluation model and a prediction model using a dataset other than the 'TILE' dataset.
FIG. 11 shows the experimental results of comparing the performance of an evaluation device using both an evaluation model and a prediction model with the performance of an existing evaluation device.
FIG. 12 shows an example illustrating the overall process in which an evaluation device 300 further receives flanking sequence information 140 and evaluates the efficiency of RNA interference.
FIG. 13 shows the results of analyzing the efficiency of RNA interference according to the number of adenine and uracil residues (AUs) in the flanking 2nt of the downstream.
FIG. 14 shows the experimental results of comparing the performance of an improved evaluation model with the performance of the evaluation model.
FIG. 15 shows the results of comparing the performance of an improved evaluation model with other evaluation models.
FIG. 16 shows the overall experimental process for confirming whether the efficiency of RNA interference evaluated by the improved evaluation model and the evaluation model are consistent with the experimental results in actual cells.
FIG. 17 shows the results of evaluating the efficiency of RNA interference by the improved evaluation model and the evaluation model, and the results of knock-down (KD) for each gene.
FIG. 18 shows an example illustrating the configuration of an evaluation device 600 for evaluating the efficiency of shRNA used for RNA interference.

### [Modes of the Invention]

The technology described below may have various modifications and various embodiments, and specific embodiments are illustrated in the drawings and described in detail. However, this is not intended to limit the technology described below to specific embodiments, and it should be understood that all modifications, equivalents, or substitutes included in the spirit and scope of the technology described below are included.

Although the terms, 'first,' 'second,' 'A,' 'B,' and the like may be used to describe various components, these components are not limited by these terms, and are only used to distinguish one component from another. For example, without departing from the scope of the technology described below, a first component may be referred to as a second component, and similarly, a second component may also be referred to as a first component. The term 'and/or' includes any combination of a plurality of relevant items that are described or any item among a plurality of relevant items that are described.

As used herein, singular expressions should be construed to include plural expressions unless the context clearly dictates otherwise, and the term "include" and the like should be understood to mean the presence of a stated feature, number, step, operation, component, part, or a combination thereof, but not to exclude the presence or addition of one or more other features, numbers, steps, operations, components, parts, or combinations thereof.

Before providing a detailed description of the drawings, it should be made clear that the division of components herein is only a division based on the main function of each component. In other words, two or more components to be described below may be combined into one component, or one component may be divided into two or more components with more detailed functions. In addition to its own main function, each component to be described below may additionally perform some or all of the functions of other components, and of course, some of the main functions of each component may be exclusively performed by other components.

**In** addition, in carrying out a method or operation method, each process constituting the method may occur in a different order from the stated order unless the context clearly states a specific order. In other words, each process may occur in the same order as the stated order, may be performed substantially simultaneously, or may be performed in the opposite order.

Before explaining the technology described below, some necessary terms are described.

In the technology described below, RNA interference refers to a process in which RNA molecules block gene expression.

In the technology described below, short hairpin RNA (shRNA) is one of the methods of RNA interference. This method refers to a process in which cells are transformed using plasmids or the like to be able to produce shRNA, and then the cells themselves produce miRNA or the like to perform RNA interference.

In the technology described below, primary shRNA (pri-shRNA) refers to the product that is first transcribed by RNA polymerase in a transformed cell to perform RNA interference using the shRNA method. Pri-shRNA is cleaved within the cell nucleus together with Drosha and other proteins to become pre-shRNA.

In the technology described below, Drosha refers to an enzyme that cleaves pri-shRNA in the nucleus to produce pre-shRNA.

In the technology described below, the learning model refers to a machine learning model, and the machine learning model may be various types of models. For example, a machine learning model may be a decision tree, a random forest (RF), a K-nearest neighbors (KNN), Naive Bayes, a support vector machine (SVM), an artificial neural network (ANN), or the like

The ANN may be a deep neural network (DNN), which may include a convolutional neural network (CNN), a recurrent neural network (RNN), a restricted Boltzmann machine (RBM), a deep belief network (DBN), a generative adversarial network (GAN), a relation network (RL), and the like. For convenience of explanation, the following explanation focuses on the CNN model.

The CNN model may consist of a convolution layer, a pooling layer, a dropout layer, a batch normalization layer, a flatten layer, and a fully connected layer (or dense layer).

The convolution layer extracts features from input information and creates a feature map therefrom. The pooling layer extracts the largest value (max pooling layer) or the average value (average pooling layer) among the values of the feature map to reduce the size of the feature map created by the convolution layer or to emphasize specific data. The dropout layer utilizes only a part of the neural network model during the training process to prevent overfitting in the deep learning model. The batch normalization layer normalizes each batch during the training process to prevent overfitting in the deep learning model. The flatten layer transforms the features of the extracted data into a single dimension. The fully connected layer connects all nodes of each layer and ultimately determines the classification to which the input data belongs. The SoftMAX function may also be used in this process.

First, the overall process for determining the efficiency of shRNA used for RNA interference is described.

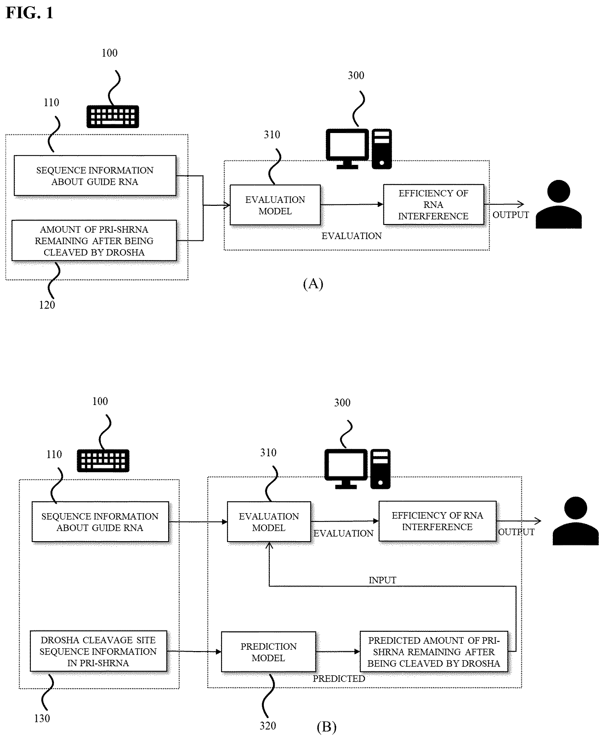

FIG. 1 shows an example illustrating the overall process for determining the efficiency of shRNA used for RNA interference.

In FIG. 1, an evaluation device 300 may receive data through an input device 100 and evaluate the efficiency of shRNA used for RNA interference through this data.

The evaluation device 300 may use only an evaluation model 310 as in FIG. 1A, or may use both an evaluation model 310 and a prediction model 320 as in FIG. 1B.

The efficiency of shRNA used for RNA interference, which is evaluated by the evaluation device 300, may include information about the extent to which RNA interference occurs by the shRNA method.

In FIG. 1, the data received by the evaluation device 300 is 'sequence information about guide RNA used for RNA interference' 110 (hereinafter referred to as 'guide RNA sequence information') and 'information about the amount of pri-shRNA remaining after being cleaved by Drosha' 120 (hereinafter referred to as 'pri-shRNA amount information') as shown in FIG. 1A. Alternatively, as shown in FIG. 1B, 'Drosha cleavage site sequence information in pri-shRNA' 130 (hereinafter referred to as 'Drosha cleavage site sequence information') may be received instead of the 'information about the amount of pri-shRNA' 120.

The 'sequence information of guide RNA used for RNA interference' 110 used as input data refers to the sequence information of guide RNA used for RNA interference. The sequence information may be 22 nt (nucleotide)-long information.

The 'amount of pri-shRNA remaining after being cleaved by Drosha' 120 used as input data may refer to the amount of pri-shRNA produced by the transformed cell and remaining after being cleaved by Drosha. As this information, an actually measured value may be used as shown in FIG. 1A, or a value predicted by a prediction model may be used as shown in FIG. 1B.

The 'Drosha cleavage site sequence information' 130 used as input data may include sequence information near a site actually cleaved by Drosha in the pri-shRNA. This may include: information concatenating a sequence from 27 nt (nucleotide) upstream to 29 nt downstream based on the 5p Drosha cleavage site in the pri-shRNA (5p Drosha cleavage site sequence) and a sequence from 31 nt upstream to 25 nt downstream based on the 3p Drosha cleavage site (3p Drosha cleavage site sequence); and information about reversing the 3p Drosha cleavage site sequence and concatenating the resulting sequence to the 5p cleavage site sequence.

The 'amount of pri-shRNA remaining after being cleaved by Drosha' 120 or 'the sequence near the cleavage site of Drosha in pri-shRNA' 130 used as input data may be related to the efficiency of shRNA. Specifically, the amount of pri-shRNA may be related to the cleavage efficiency of Drosha. The specific details are described below.

In FIG. 1, the evaluation model 310 outputs a result value for evaluating the efficiency of shRNA used for RNA interference, based on the received 'guide RNA sequence information' 110 and 'pri-shRNA amount information' 120 data.

The evaluation model may be a learning model trained with existing data to evaluate the efficiency of shRNA used for RNA interference.

In FIG. 1, the prediction model 320 receives 'Drosha cleavage site sequence information' 130 and outputs a result value for predicting 'the amount of pri-shRNA remaining after the pri-shRNA is cleaved by Drosha.'

The prediction model may be a learning model trained with existing data to predict 'the amount of pri-shRNA remaining after pri-shRNA is cleaved by Drosha.'

The prediction model may be stored and used in an evaluation device such as an evaluation model, but may also be stored and used in a prediction device different from the evaluation device (not shown).

In a method of analyzing the efficiency of shRNA using an evaluation model, the reason for using 'the amount of pri-shRNA remaining after being cleaved by Drosha' is that 'the amount of pri-shRNA remaining after being cleaved by Drosha' represents the cleavage efficiency of Drosha, which is negatively correlated with 'the efficiency of shRNA.'

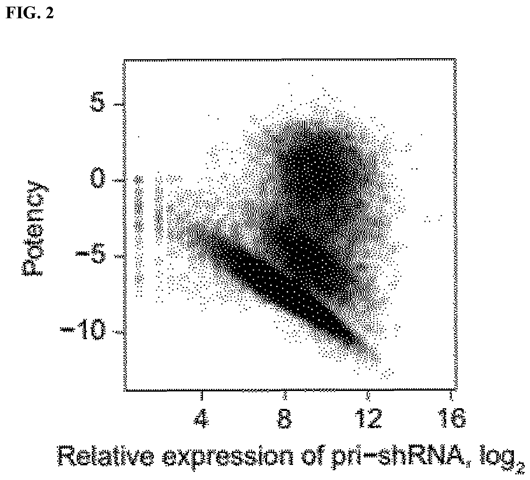

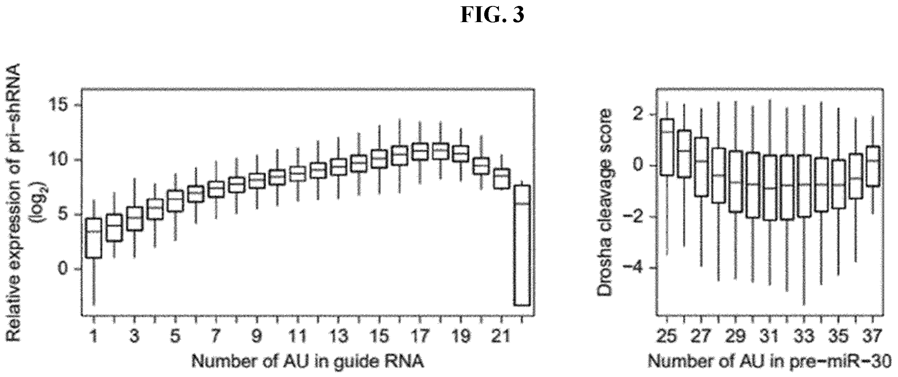

Hereinafter, experimental results (FIGS. 2 and 3) confirming that the amount of pri-shRNA and the efficiency of shRNA are negatively correlated are described.

FIG. 2 shows the experimental results illustrating that the 'amount of pri-shRNA' and the 'efficiency of shRNA' are negatively correlated.

The graph in FIG. 2 illustrates the analysis using RNA sequencing data of 'TILE' data among different types of data described below.

In the graph in FIG. 2, the X-axis shows the relative amount of pri-shRNA in the cell, and the Y-axis shows the results of measuring the efficiency of RNA interference.

As can be seen in the graph in FIG. 2, as the amount of pri-shRNA increased, the efficiency of RNA interference decreased. Conversely, it can be seen that as the amount of pri-shRNA decreased, the efficiency of RNA interference increased.

When the results shown in FIG. 2 are analyzed, it can be confirmed that the amount of pri-shRNA and the efficiency of RNA interference are negatively correlated.

FIG. 3 shows the experimental results illustrating that the 'amount of pri-shRNA' and the 'efficiency of pri-shRNA being cleaved by Drosha' are negatively correlated.

The X-axis in the graphs of FIGS. 3A and 3B shows the number of AU (adenine and uracil) residues in guide RNA and pre-shRNA, respectively, and the Y-axis in the graph of FIG. 3A shows the relative amount of pri-shRNA, whereas the graph of FIG. 3B shows the degree of pri-shRNA cleavage by Drosha.

As can be seen in the graph of FIG. 3A, the relationship between the 'number of AU residues in guide RNA' and the amount of pri-shRNA is an inverted U-shaped relationship.

As can be seen in the graph of FIG. 3B, the relationship between the 'number of AU residues in pre-miR-30' and 'the cleavage efficiency of Drosha' is an upright U-shaped relationship. The pre-miR-30 is one type of pre-shRNA and employs miR-30 as a backbone.

When the results shown in FIGS. 3A and 3B are analyzed, it can be confirmed that the amount of pri-shRNA and the cleavage efficiency of Drosha are negatively correlated with each other. It can be confirmed that as more pri-shRNA was cleaved by Drosha, the amount of pre-shRNA and mature-shRNA increased, and through this, the efficiency of RNA interference increased.

The conclusion derived from the analysis illustrated in FIG. 3 is consistent with the conclusion obtained in FIG. 2 that 'the amount of pri-shRNA and the efficiency of RNA interference are negatively correlated.'

The present inventor implemented an evaluation model 310 evaluating the efficiency of shRNA by utilizing the fact that the amount of 'pri-shRNA remaining after being cleaved by Drosha' is negatively correlated with 'shRNA efficiency.'

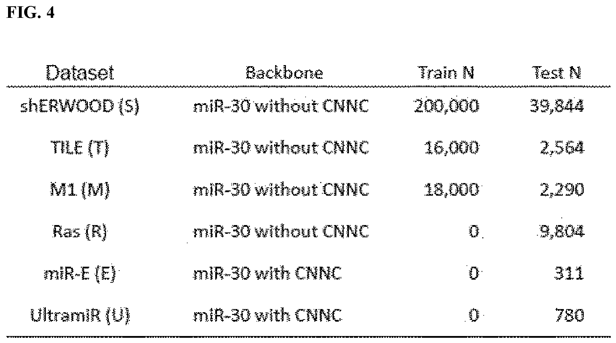

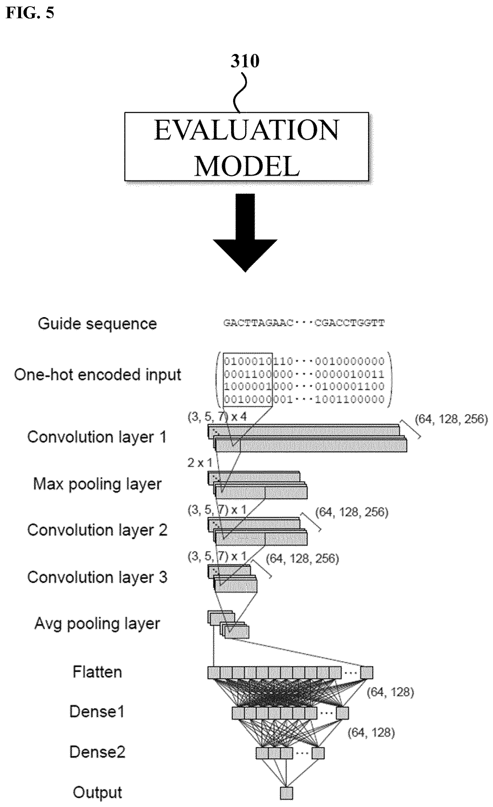

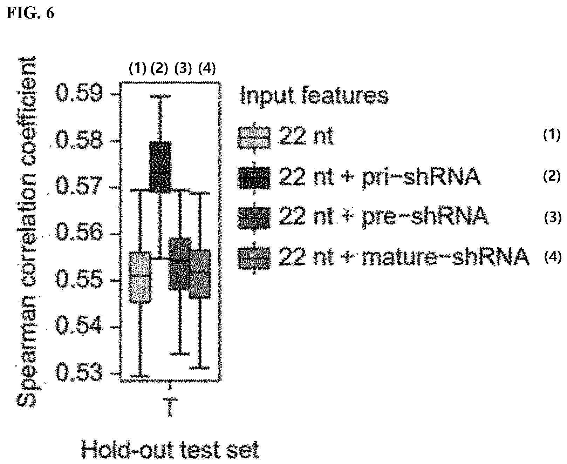

Hereinafter, the data used to build an evaluation model 310 (FIG. 4), the structure of the model (FIG. 5), and the results of evaluating the performance (FIG. 6) are described.

FIG. 4 shows an example illustrating the types and number of pieces of training or test data used for implementing the evaluation model 310.

The data may also be used to implement the prediction model 320 described below.

As the above-described data, data used in a previously published paper was used. Among the data, shERWOOD may be found in Knott, Simon RV, et al., "A computational algorithm to predict shRNA potency," Molecular Cell 56.6 (2014): 796-807; TILE in Fellmann, Christof, et al., "Functional identification of optimized RNAi triggers using a massively parallel sensor," Molecular Cell 41.6 (2011): 733-746, Ras in Yuan, Tina L., et al., "Development of siRNA Payloads to Target KRAS-Mutant CancerRNAi Therapy for KRAS-Mutant Cancer," Cancer Discovery 4.10 (2014): 1182-1197, and M1/mirR-E in Pelossof, Raphael, et al., "Prediction of potent shRNAs with a sequential classification algorithm," Nature Biotechnology 35.4 (2017): 350-353.

The types of data sets that may be used as the above-described training data include shERWOOD (S), TILE (T), and M1 (M). At this time, 200,000 pieces of shERWOOD data, 16,000 pieces of TILE data, and 18,000 pieces of M1 data may be used as training data.

The types of data sets that may be used as the above-described test data include shERWOOD (S), TILE (T), M1 (M), RasshRNA, miR-EshRNA, and UltramiR(U). At this time, 39,844 pieces of shERWOOD data, 2,564 pieces of TILE data, 2,290 pieces of M1 data, 9,804 pieces of Ras data, 311 pieces of miR-E data, and 780 pieces of UltramiR data may be used as test data.

For the training data and test data, miR-30 was used as the backbone. Among these, shERWOOD, TILE, M1, and Ras used miR-30 that included no information about the CNNC sequence, but miR-E and UltramiR used miR-30 that included information about the CNNC sequence.

In the CNNC, C means a cytosine residue, and N means any nucleotide, i.e., A, T, C, or G. CNNC refers to a sequence that, together with the UG motif, helps shRNA and serine/arginine-rich splicing factor 3 (SRSF3) to attach to pre-shRNA, thereby promoting the cleavage of pri-shRNA into pre-shRNA.

FIG. 5 shows an example in which the structure of an evaluation model 310 implemented as a learning model is implemented as a convolution neural network (CNN).

The evaluation model 310 may consist of convolution layers and fully connected layers. The convolution layers of the evaluation model may include three convolution layers, a max pooling layer between the first and second convolution layers, and an average pooling layer after the last convolution layer. The fully connected layers of the evaluation model may include a flatten layer and two dense layers.

The sequence of the guide RNA that the evaluation model 310 of FIG. 5 receives consists of a series of nucleotides. The evaluation model receives the sequence of the guide RNA in a one-hot vector-encoded form according to the type of nucleotide (A/T/G/C). A matrix of a size of 22 X 4, formed by one-hot vector encoding of 22 nt-long guide RNA, may be constructed and input to the evaluation model.

The evaluation model 310 may also receive further input data using a concatenation layer.

The output values of the convolution layers of FIG. 5 may be batch-normalized, and then activated using an exponential linear unit (ELU) or rectified exponential linear unit (RELU).

The size of the filter used in the first convolution layer may be 3X4, 5X4, or 7X4, and the number of filters may be 64, 128, or 256.

The size of the filter used in the second and third convolution layers may be 3X1, 5X1, or 7X1, and the number of filters may be 64, 128, or 256.

The size of the filter used in the max pooling layer of FIG. 5 may be 2X1, and the stride may be 2.

The size of the first and second dense layers in FIG. 5 may be 64 or 128. After the second dense layer, a layer that linearly transforms the previous result value and outputs the regression result value may be added in order to output values for the efficiency of shRNA used for RNA interference.

FIG. 6 shows the experimental results confirming the performance of an evaluation model 310 according to data that may be further input.

In the graph of FIG. 6, "T" on the X-axis represents the "TILE" data set as information used to test the evaluation model, and the Y-axis represents the Spearman correlation coefficient.

As shown in the graph in FIG. 6, it can be confirmed that performance was higher when the information about the amount of pri-shRNA (22nt + pri-shRNA, second from the left in the graph, (2)) was used together with the sequence information of the guide RNA than when only the sequence information about guide RNA (22nt, leftmost in the graph, (1)) was used as input data (input feature).

In addition, it can be confirmed that the models that used the amount of pre-shRNA or the amount of mature-shRNA instead of the amount of pri-shRNA as input data (22nt + pre-sRNA (3) or 22nt + mature-shRNA (4), 3rd and 4th from the left) exhibited almost no difference in performance from the model that used only the information about the guide RNA.

According to the experimental results shown in FIG. 6, it can be confirmed that the evaluation model that used not only the sequence information about guide RNA but also the information about the amount of pri-shRNA exhibited the best performance.

The information about the shRNA to be evaluated may not include the information about the 'actually measured amount of pri-shRNA.' Therefore, the present inventor constructed a prediction model 320 predicting the 'amount of pri-shRNA' based on the given information.

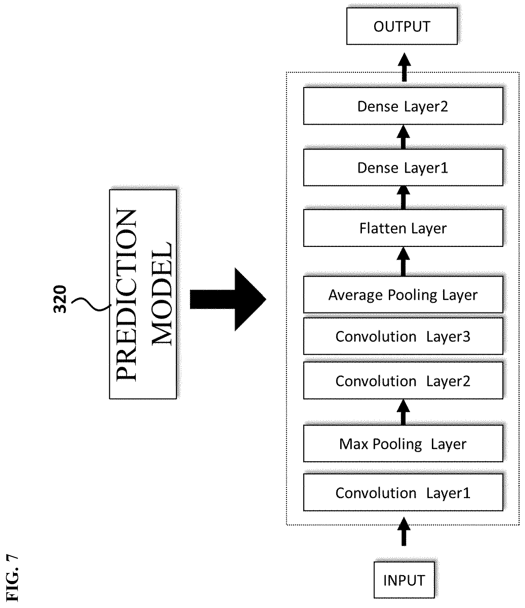

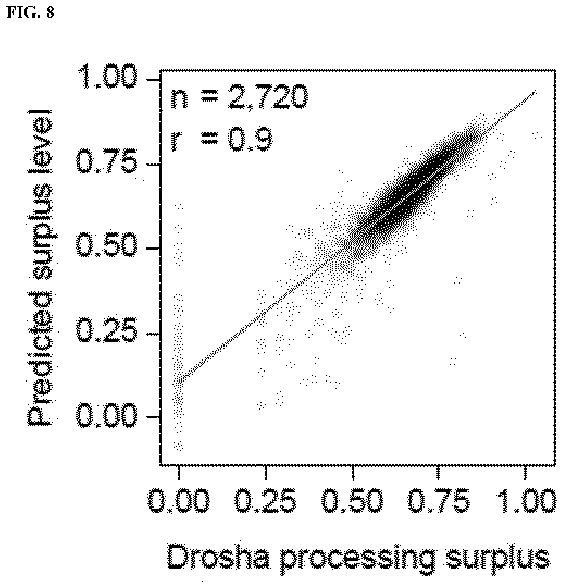

Hereinafter, the structure (FIG. 7) and performance (FIG. 8) of the prediction model 320 are described.

FIG. 7 shows an example in which the structure of a prediction model 320 implemented as a learning model is implemented as a CNN.

The prediction model 320 may consist of convolution layers and fully connected layers. The convolution layers of the evaluation model may include three convolution layers, a max pooling layer located between the first and second convolution layers, and an average pooling layer located after the last convolution layer. The fully connected layers of the prediction model may include a flatten layer and two dense layers.

The prediction model 320 of FIG. 7 receives a Drosha cleavage site sequence as input.

The Drosha cleavage site sequence may include: information concatenating a sequence from 27 nt (nucleotide) upstream to 29 nt downstream based on the 5p Drosha cleavage site in the pri-shRNA (5p Drosha cleavage site sequence) and a sequence from 31 nt upstream to 25 nt downstream based on the 3p Drosha cleavage site (3p Drosha cleavage site sequence); and information about reversing the 3p Drosha cleavage site sequence and concatenating the resulting sequence to the 5p cleavage site sequence.

The Drosha cleavage site sequence consists of a series of nucleotides. The Drosha cleavage site sequence is one-hot vector-encoded according to the type of nucleotide (A/T/G/C).

The output values of the convolution layers of FIG. 7 may be batch-normalized, and then activated using an ELU or RELU.

The size of the filter used in the first convolution layer may be 9X9, and the number of filters may be 128, 256, or 512.

The size of the filter used in the second and third convolution layers may be 3X1, 5X1, or 7X1, and the number of filters may be 128, 256, or 512.

The size of the filter used in the max pooling layer of FIG. 7 may be 2X1, and the stride may be 2.

The size of the first and second dense layers of FIG. 7 may be 64 or 128.

The output values of the dense layers of FIG. 7 may be batch-normalized, and then activated using an ELU or RELU.

After the second dense layer, a layer that linearly transforms the result values of the previous layer and outputs the regression result values may be added to output the predicted value of the amount of pri-shRNA remaining after being cleaved by Drosha.

FIG. 8 shows the experimental results of evaluating the performance of the constructed prediction model 320.

In FIG. 8, the X-axis represents the actual 'amount of pri-shRNA remaining after being cleaved by Drosha,' and the Y-axis represents the 'amount of pri-shRNA remaining after being cleaved by Drosha' predicted by the prediction model.

When the results in FIG. 8 are analyzed, it can be confirmed that the correlation coefficient (R value) between the actual remaining amount and the amount predicted by the prediction model was 0.9. Therefore, the predicted value of the prediction model 320 matches the actual value well.

The present inventor confirmed the performance of the method for predicting the efficiency of shRNA using the evaluation model 310 and the prediction model 320.

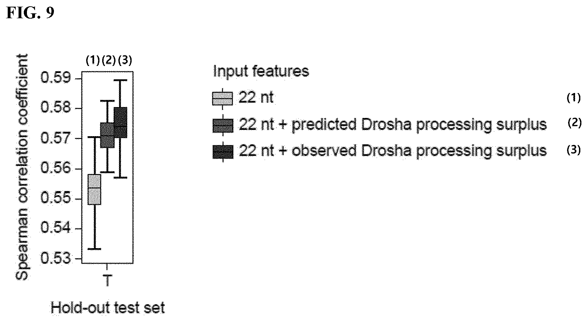

Hereinafter, the experimental results of evaluating performance are described (FIGS. 9, 10, and 11).

FIG. 9 shows the experimental results of evaluating the performance of the shRNA efficiency prediction method using an evaluation model 310 and a prediction model 320.

In FIG. 9, the 'T' on the X-axis represents the type of data used for the test, which is the TILE data set, and the Y-axis represents the Spearman correlation coefficient.

As shown in FIG. 9, the performance of the model that used only the sequence information of the guide RNA as the input data (input feature) (22nt, leftmost in the graph, (1)) was the lowest. On the other hand, the model in which the actually measured amount of pri-shRNA and the guide RNA sequence information were input (22nt + observed Drosha processing surplus, (3), rightmost in the graph) exhibited the best performance. In addition, the model that used the value of the pri-shRNA predicted by the prediction model instead of the actually measured value as input data (22nt + predicted Drosha processing surplus, (2) in the middle of the graph) also exhibited better performance than the model that used only the sequence information of guide RNA.

When the results shown in FIG. 9 are analyzed, it can be seen that the method of evaluating shRNA efficiency using both the evaluation model and the prediction model exhibited higher performance than the case in which only the sequence information of guide RNA was used, and there was no significant difference from the case in which the actually measured amount of pri-shRNA was used.

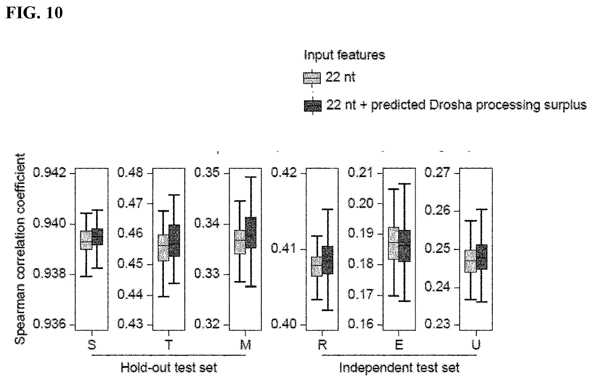

FIG. 10 shows the experimental results of evaluating the performance of a shRNA efficiency prediction method using an evaluation model 310 and a prediction model 320 using a dataset other than the 'TILE' dataset.

In FIG. 10, the X-axis represents the type of data used for the test (holdout and independent), and the Y-axis represents the Spearman correlation coefficient. The letters S, T, M, R, E, and U written on the X-axis represent the types of data shown in FIG. 4.

As shown in FIG. 10, it can be confirmed that the model in which the predicted values from the prediction model were further input in addition to the guide RNA sequence information (right side of each graph) exhibited better performance than the model that simply used the guide RNA sequence information as input data (left side of each graph).

When the results shown in FIG. 10 are analyzed, it can be confirmed that the performance of the evaluation device that determined the efficiency of shRNA used for RNA interference using both the evaluation model and the prediction model was better than that of the evaluation device that determined the efficiency of shRNA used for RNA using only the guide RNA sequence information, even in the cases in which test data sets other than the 'TILE' data set were used as test data.

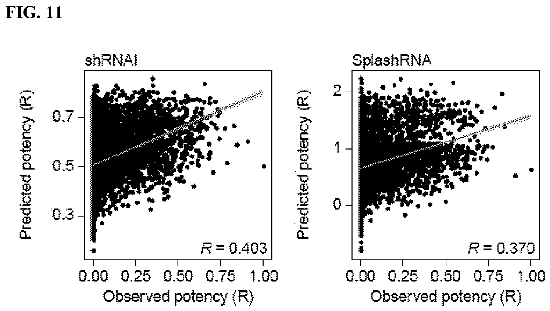

FIG. 11 shows the experimental results of comparing the performance of an evaluation device using both an evaluation model 310 and a prediction model 320 with the performance of an existing evaluation device.

In FIG. 11, shRNAI on the left represents the evaluation device using both the evaluation model and the prediction model, and SplashRNA on the right represents the existing evaluation device using only the sequence information of guide RNA.

In FIG. 11, the X-axis of each graph represents the actually observed efficiency of RNA interference, and the Y-axis represents the efficiency of RNA interference predicted by the model.

As shown in FIG. 11, the correlation coefficient of the evaluation device using both the evaluation model and the prediction model was 0.403, while the correlation coefficient of the existing evaluation device was 0.370.

When the results shown in FIG. 11 are analyzed, it can be confirmed that the performance of the evaluation device using both the evaluation model and the prediction model was about 9% higher than the performance of the existing evaluation device.

The above-described evaluation device 300 may evaluate the efficiency of RNA interference by further receiving flanking sequence information along with guide RNA sequence information.

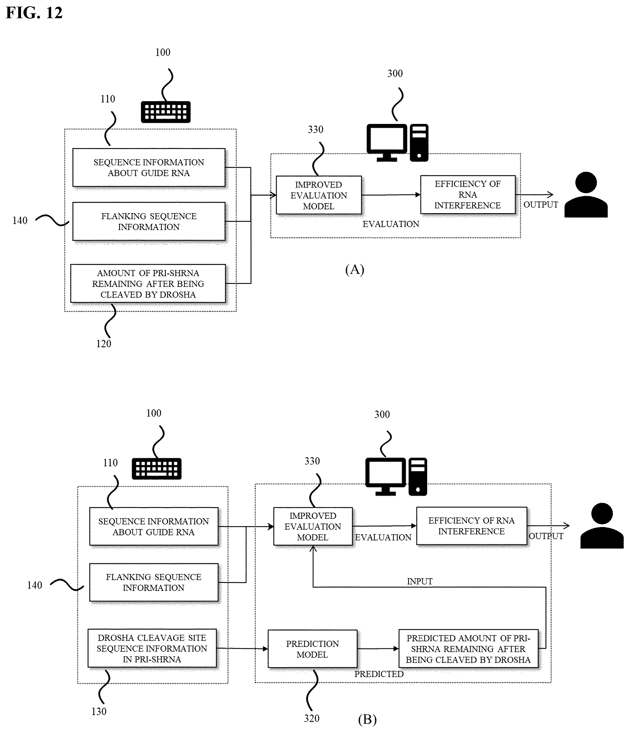

FIG. 12 shows an example illustrating the overall process in which an evaluation device 300 further receives flanking sequence information 140 and evaluates the efficiency of RNA interference.

The evaluation device 300 may further receive flanking sequence information 140 in addition to guide RNA sequence information 110 and pri-shRNA amount information 120 (FIG. 12A). Similarly, the evaluation device 300 may also employ a prediction model 320 to predict the pri-shRNA amount information using Drosha cleavage site sequence information 130 in pri-shRNA and then use it (FIG. 12B).

The evaluation device 300 may employ an improved evaluation model 330 to utilize flanking sequence information 140. The improved evaluation model 330 may be a model that further receives flanking sequence information 140 and evaluates the efficiency of RNA interference. The improved evaluation model 330 may be a model based on the evaluation model 310. In one embodiment, the improved evaluation model 330 may be a model constructed by fine-tuning the evaluation model 310.

The additionally input flanking sequence information 140 may include sequence information upstream and downstream of guide RNA. As an example, the flanking sequence information 140 may include sequence information of 14 nt upstream and 14 nt downstream of guide RNA. In other words, the evaluation device 300 may evaluate the efficiency of RNA interference based on 50 nt sequence information including 22 nt guide RNA sequence information and 28 nt flanking sequence information (14 nt x 2).

Hereinafter, the process in which the present inventor constructed an improved evaluation model 330 and the experimental results of evaluating the performance of the constructed improved evaluation model 330 will be described.

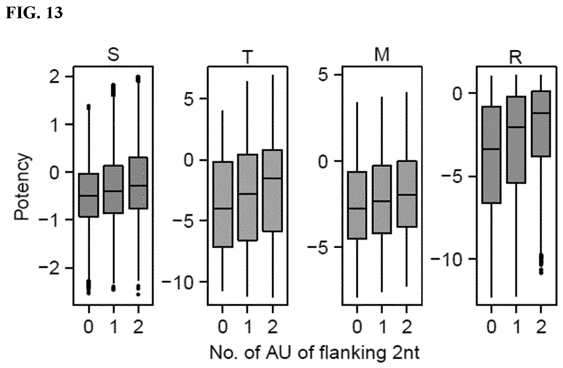

FIG. 13 shows the results of analyzing the efficiency of RNA interference according to the number of AU residues downstream of the flanking 2nt. The data sets used for the analysis are the data sets shown in FIG. 4 (shERWOOD (S), TILE (T), M1 (M), and Ras (R)).

When the results shown in FIG. 13 are analyzed, it can be confirmed that the efficiency of RNA interference increased as the number of AU residues downstream of the flanking 2nt increased. This can be confirmed in each of all data sets (S, T, M, and R).

Through FIG. 13, it can be confirmed that RNA interference efficiency is affected not only by the sequence information about guide RNA but also by the flanking sequence information. In other words, it can be confirmed that RNA interference efficiency can be evaluated more accurately by using not only the guide RNA sequence information but also the flanking sequence information.

Based on this fact, the present inventor constructed a model capable of using not only the guide RNA sequence information but also the flanking sequence information. Specifically, the present inventor constructed an improved evaluation model 330 capable of further receiving the flanking sequence information based on the evaluation model 310 receiving the guide RNA sequence information and the pri-shRNA amount information. Specifically, the improved evaluation model 330 is a model that has been constructed by adjusting the parameters of the evaluation model 310.

The improved evaluation model is capable of receiving not only the guide RNA sequence information but also the flanking sequence information. Specifically, the improved evaluation model is capable of receiving information about 22nt, which is the guide RNA sequence information, and information about 28nt (14nt x 2), which is the flanking sequence information. In other words, the improved evaluation model is also capable of receiving 50 nt base sequence information as an input.

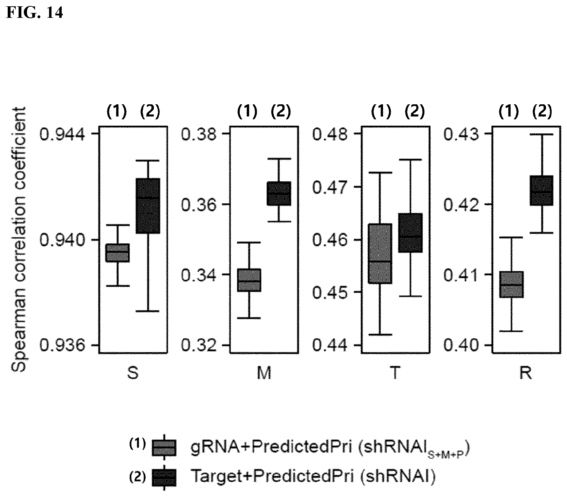

FIG. 14 shows the experimental results of comparing the performance of an improved evaluation model with the performance of the evaluation model.

In FIG. 14, the bar on the left of each graph shows the results of evaluating the performance of the evaluation model (shRNAI_{S+M+P}) that uses only the guide RNA sequence information and the predicted pri-shRNA amount information. In other words, it is the evaluation model 310 in FIG. 1.

In FIG. 14, the bar on the right of each graph shows the results of evaluating the performance of the improved evaluation model (shRNAI) that uses the flanking sequence information as additional input data. In other words, it is the improved evaluation model 330 in FIG. 12.

Considering the results shown in FIG. 14, it can be confirmed that the performance of the model that uses the target sequence information (guide RNA sequence information + flanking sequence information) and the predicted pri-shRNA amount information is higher than the performance of the evaluation model that simply uses the guide RNA sequence information and the predicted pri-shRNA amount information. In other words, it can be confirmed that the performance of the improved evaluation model 330 that further uses the flanking sequence information is higher than the performance of the evaluation model 310.

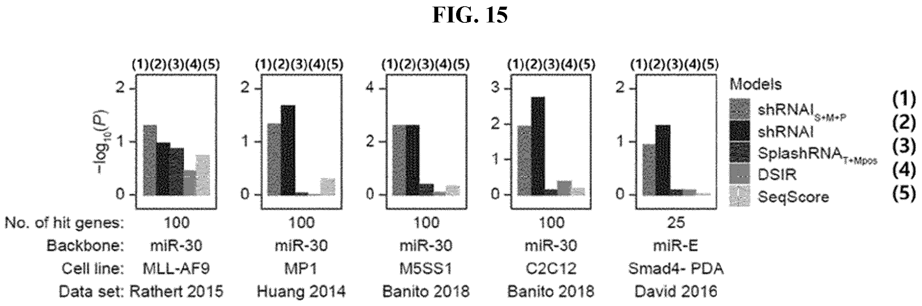

FIG. 15 shows the results of comparing the performance of an improved evaluation model with other evaluation models. Specifically, the results were obtained by comparing the performance of the improved evaluation model (shRNAI, (1)) and the evaluation model (shRNAI_{S+M+P}, (2)) with the control groups (SplashRNA_{T+Mpos} (3), DSIR (4), and SeqScore (5)). The experiment was conducted using a plurality of data sets. The backbones and cells used in the data sets were different.

Considering the results shown in FIG. 15, it can be confirmed that the performance of the improved evaluation model (shRNAI) and the evaluation model (shRNAI_{S+M+P}) is higher than that of the control groups (SplashRNA_{T+Mpos}, DSIR, and SeqScore). In addition, in some data sets (Huang2014, Banito2018, and David 2016), the performance of the improved evaluation model (shRNAI) is higher than that of the evaluation model (shRNAI_{S+M+P}).

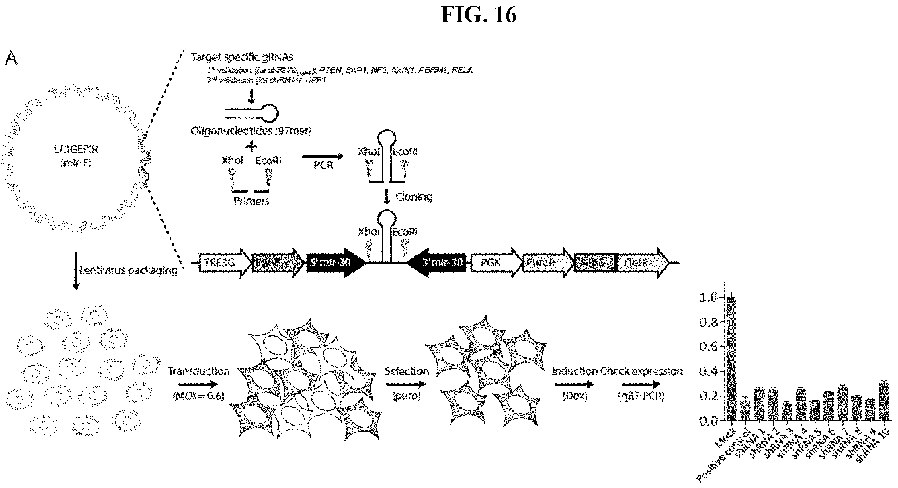

FIG. 16 shows the overall experimental process for confirming whether the efficiency of RNA interference evaluated by the improved evaluation model and the evaluation model are consistent with the experimental results in actual cells.

The target genes used to evaluate the performance of the evaluation model (shRNAI_{S+M+P}) were PTEN, BAP1, NF2, AXN1, PBPM1, and RELA. The target gene used to evaluate the performance of the improved evaluation model (shRNAI) was UPF1. The vector used for transformation was mlr-E (LT3GEPIR), and quantitative reverse transcription polymerase chain reaction (qRT-PCR) was used to confirm whether RNA interference actually occurred.

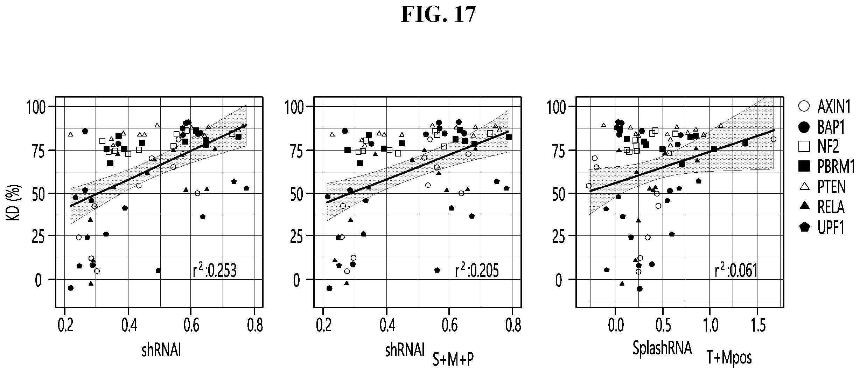

FIG. 17 shows the results of evaluating the efficiency of RNA interference by the improved evaluation model and the evaluation model using the experimental method shown in FIG. 16, and the results of knock-down (KD) for each gene.

The (SplashRNA_{T+Mpos}) model was used as the control group of the improved evaluation model (shRNAI) and the evaluation model (shRNAI_{S+M+P}).

The r² of the control group (SplashRNA_{T+Mpos}) was 0.061, while the r² of the improved evaluation model (shRNAI) and the evaluation model (shRNAI_{S+M+P}) was 0.253 and 0.205, respectively, which were higher. In particular, the r² of the improved evaluation model (shRNAI) was the highest.

Through FIG. 17, it can be confirmed that the performance of the improved evaluation model (shRNAI) and the evaluation model (shRNAI_{S+M+P}) is higher than that of the control group (SplashRNA_{T+Mpos}). In particular, it can also be confirmed that the performance of the improved evaluation model (shRNAI) is higher than that of the evaluation model (shRNAI_{S+M+P}).

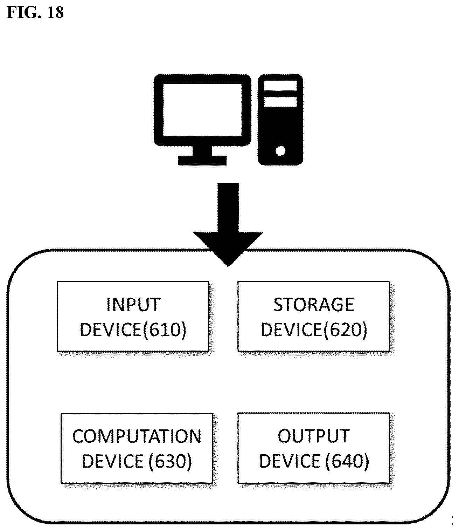

Hereinafter, an evaluation device evaluating the efficiency of shRNA used for RNA interference is described through FIG. 18.

FIG. 18 shows an example illustrating the configuration of an evaluation device 600 for evaluating the efficiency of shRNA used for RNA interference.

The evaluation device 600 corresponds to the above-described evaluation device (300 of FIG. 1).

The evaluation device 600 may be implemented in various physical forms, such as a PC, a laptop, a smart device, a server, and a data processing-only chipset.

The evaluation device 600 may include an input device 610, a storage device 620, and a computation device 630.

The evaluation device may further include an output device 640.

The input device 610 of FIG. 18 may include an interface device (keyboard, mouse, touch screen, etc.) that receives a certain command or data as an input.

The input device 610 corresponds to the above-described input device (100 of FIG. 1).

The input device 610 may include a communication device that receives and transmits certain information through a wired or wireless network.

The input device 610 may include a component receiving information through a separate storage device (USB, CD, hard disk, etc.).

The input device 610 may receive the input data through a separate measuring device or a separate DB.

The input device 610 may also receive sequence information about guide RNA and a value for the amount of pri-shRNA remaining after being cleaved by Drosha.

The input device 610 may also receive 'flanking sequence information of the Drosha cleavage site in pri-shRNA' instead of information about the amount of 'pri-shRNA remaining after being cleaved by Drosha.'

The input device 610 may receive flanking sequence information.

The storage device 620 of FIG. 18 may store information from the input device 610.

The storage device 620 may store an evaluation model (310 in FIG. 1) analyzing the efficiency of shRNA used for RNA interference or a prediction model (320 in FIG. 1) predicting the amount of pri-shRNA. Alternatively, the storage device 620 may store an improved evaluation model (330 in FIG. 12).

The storage device 620 may store the output values of the evaluation model and the prediction model.

The storage device 620 may store training data used by a learning model.

The computation device 630 of FIG. 18 may analyze the efficiency of shRNA used for RNA interference based on the output values after inputting the 'guide RNA sequence information' and 'the amount of pri-shRNA remaining after being cleaved by Drosha,' received from the input device 610, into the evaluation model.

The computation device 630 may analyze the efficiency of shRNA used for RNA interference by inputting the 'predicted amount of pri-shRNA,' output by the prediction model, into the evaluation model, instead of the 'the amount of pri-shRNA remaining after being cleaved by Drosha.'

The computation device 630 may further input 'flanking sequence information' into the evaluation model and then evaluate the efficiency of shRNA used for RNA interference based on the output values of the evaluation model. In other words, the computation device 630 may evaluate the efficiency of shRNA used for RNA interference using the improved evaluation model.

The output device 640 of FIG. 18 may be a device outputting certain information.

The output device 640 may output interfaces required for data processing, input data, analysis results, and the like.

The output device 640 may be implemented in various physical forms, such as a display, a document output device, a communication device, and the like.

In addition, the method of evaluating the efficiency of candidate shRNA used for RNA interference as described above may be implemented as a program (or application) including an executable algorithm that can be executed on a computer. The program may be provided by being stored on a transitory or non-transitory computer readable medium.

A non-transitory readable medium refers to a medium that stores data semi-permanently and allows the data to be read by a device, rather than a medium that stores data for a short period of time, such as a register, cache, or memory. Specifically, the various applications or programs described above may be stored and provided in a non-transitory readable medium, such as a CD, DVD, hard disk, Blu-ray disk, USB, memory card, read-only memory (ROM), programmable read only memory (PROM), erasable PROM (EPROM), or electrically EPROM (EEPROM), or flash memory.

A transitory readable medium refers to various RAMs such as a static RAM (SRAM), dynamic RAM (DRAM), synchronous DRAM (SDRAM), double data rate SDRAM (DDR SDRAM), enhanced SDRAM (ESDRAM), synclink DRAM (SLDRAM), and direct rambus RAM (DRRAM).

The present embodiments and the drawings attached to the present specification only clearly show a part of the technical idea included in the above-described technology, and it will be obvious that all modified examples and specific embodiments that can be easily inferred by a person skilled in the art within the scope of the technical idea included in the specification and drawings of the above-described technology are included in the scope of the rights of the above-described technology.

## Claims

1. A method of evaluating the efficiency of a candidate short hairpin RNA (shRNA) used for RNA interference, the method comprising:
a step in which an evaluation device receives 'sequence information about a candidate guide RNA used for RNA interference' and 'information about the amount of primary short hairpin RNA (pri-shRNA) remaining after being cleaved by Drosha';
a step in which the evaluation device inputs the received 'sequence information about the guide RNA' and 'information about the amount of pri-shRNA remaining after being cleaved' into an evaluation model evaluating the efficiency of shRNA used for RNA interference; and
a step in which the evaluation device evaluates the efficiency of the shRNA used for RNA interference based on values output by the evaluation model.

2. The method of claim 1, wherein the evaluation model is a learning model trained to evaluate the 'efficiency of shRNA used for RNA interference' using training data, and the training data includes 'sequence information about guide RNA used for RNA interference' and 'information about the amount of pri-shRNA remaining after being cleaved by Drosha.'

3. The method of claim 1, wherein the 'information about the amount of pri-shRNA remaining after being cleaved by Drosha' in cells is information from an output value of a separate prediction model, and the prediction model is a model that receives the 'sequence information about the cleavage site of Drosha in pri-shRNA' as input data and predicts how much the pri-shRNA will remain after being cleaved by Drosha.

4. The method of claim 3, wherein the sequence information about the cleavage site input to the prediction model includes:
information concatenating a sequence from 27 nt (nucleotide) upstream to 29 nt downstream based on the 5p Drosha cleavage site in the pri-shRNA (5p Drosha cleavage site sequence) and a sequence from 31 nt upstream to 25 nt downstream based on the 3p Drosha cleavage site (3p Drosha cleavage site sequence); and
information about reversing the 3p Drosha cleavage site sequence and concatenating the resulting sequence to the 5p cleavage site sequence.

5. The method of claim 3, wherein the prediction model is a learning model trained to predict how much 'the amount of pri-shRNA remaining after being cleaved by Drosha' will be, using training data, and the training data includes 'sequence information about the cleavage site of Drosha in pri-shRNA.'

6. The method of claim 1, wherein the step of receiving includes a step of further receiving 'flanking sequence information,' the step of inputting into an evaluation model includes further inputting the 'flanking sequence information' into the evaluation model, and the flanking sequence information includes sequence information upstream and downstream of the sequence information of the guide RNA.

7. The method of claim 6, wherein the flanking sequence information includes 14nt sequence information upstream and 14nt sequence information downstream of the sequence information of the guide RNA.

8. A device for evaluating the efficiency of a candidate shRNA used for RNA interference, the device comprising:
an input device receiving sequence information about a candidate guide RNA used for RNA interference and information about the amount of pri-shRNA remaining after being cleaved by Drosha;
a storage device storing an evaluation model that evaluates the efficiency of shRNA used for RNA interference, using the guide RNA sequence information and the information about the amount of pri-shRNA remaining after being cleaved, received by the input device; and
a computation device inputting the sequence information about the guide RNA and the information about the amount of pri-shRNA remaining after being cleaved, received by the input device, into the evaluation model stored in the storage device, and then evaluating the efficiency of shRNA used for RNA interference according to values output by the evaluation model.

9. The device of claim 8, wherein the evaluation model is a learning model trained to evaluate the efficiency of shRNA used for RNA interference using training data, and the training data includes 'sequence information about guide RNA used for RNA interference' and 'information about the amount of pri-shRNA remaining after being cleaved by Drosha.'

10. The device of claim 8, wherein the 'information about the amount of pri-shRNA remaining after being cleaved by Drosha' in cells is information from an output value of a separate prediction model, and the prediction model is a model that receives the 'sequence information about the cleavage site of Drosha in pri-shRNA' as input data and predicts how much the pri-shRNA will remain after being cleaved by Drosha.

11. The device of claim 10, wherein the sequence information about the cleavage site input to the prediction model includes: information concatenating a sequence from 27 nt (nucleotide) upstream to 29 nt downstream based on the 5p Drosha cleavage site in the pri-shRNA (5p Drosha cleavage site sequence) and a sequence from 31 nt upstream to 25 nt downstream based on the 3p Drosha cleavage site (3p Drosha cleavage site sequence); and
information about reversing the 3p Drosha cleavage site sequence and concatenating the resulting sequence to the 5p cleavage site sequence.

12. The device of claim 10, wherein the prediction model is a learning model trained to predict how much 'the amount of pri-shRNA remaining after being cleaved by Drosha' will be, using training data, and the training data includes 'sequence information about the cleavage site of Drosha in pri-shRNA.'

13. The device of claim 8, wherein the input device further receives flanking sequence information,
the evaluation model is a model evaluating the efficiency of shRNA used for RNA interference, using not only sequence information about guide RNA and information about the amount of pri-shRNA remaining after being cleaved but also the flanking sequence information,
the computation device further inputs the 'flanking sequence information' into the evaluation model and then evaluates the efficiency of shRNA used for RNA interference according to values output by the evaluation model, and
the flanking sequence information includes sequence information upstream and downstream of the sequence information of the guide RNA.

14. The device of claim 13, wherein the flanking sequence information includes 14nt sequence information upstream and 14nt sequence information downstream of the sequence information of the guide RNA.

15. A computer-readable recording medium on which a program for executing the method of evaluating the efficiency of candidate shRNA used for RNA interference as recited in claim 1 on a computer is recorded.
